# EUROPEAN PATENT SPECIFICATION

(11) **EP 1 732 886 B1**
(45) Date of publication and mention of the grant of the patent: **03.03.2010**
(21) Application number: 05729986.9
(22) Date of filing: 08.04.2005
(51) Int. Cl.: C07D 207/34

(54) **POLYMORPHS OF ATORVASTATIN TERT.-BUTYLESTER AND USE AS INTERMEDIATES FOR THE PREPARATION OF ATORVASTATIN**
POLYMORPHE VON ATORVASTATIN-TERT.-BUTYLESTER UND DEREN VERWENDUNG ALS ZWISCHENPRODUKTE FÜR DIE HERSTELLUNG VON ATORVASTATIN
POLYMORPHES D'ESTER DE TERT-BUTYLE D'ATORVASTATINE ET LEUR UTILISATION EN TANT QU'INTERMEDIAIRES DANS LA PREPARATION D'ATORVASTATINE

(30) Priority: 09.04.2004 SI 200400112
(43) Date of publication of application: 20.12.2006
(73) Proprietor: KRKA, tovarna zdravil, d.d., Novo mesto, 8501 Novo mesto (SI)
(72) Inventor: STIMAC, Anton, 1000 Ljubljana (SI); ZUPET, Rok, 1000 Ljubljana (SI); GRCMAN, Marija, 8212 Velika Loka (SI); SMRKOLJ, Matej, 1411 Izlake (SI); JAKSE, Renata, 8220 Smarjeske Toplice (SI)
(74) Representative: UEXKÜLL & STOLBERG
(86) International application number: PCT/EP2005/003733
(87) International publication number: WO 2005/097742

(56) References cited:
- WO-A-02/43667
- WO-A-02/055519
- WO-A-03/016317
- LEE ET AL.: "Atorvastatin, and HMG-COA reductase inhibitor and effective lipid-regulating agent. Part II" J. LABELLED COMPOUNDS AND RADIOPHARMACEUTICALS, vol. 42, 1999, pages 129-133, XP002331696 cited in the application

## Description

### Field of the Invention

The present invention relates to new crystalline forms 1 and 2 of [R-(R*,R*)]-2-(4-fluorophenyl)-β,δ-dihydroxy-5-(1-methylethyl)-3-phenyl-4-[(phenylamino)carbonyl]-1*H*-pyrrole-1-heptanoic acid tertiary butyl ester of formula (II) 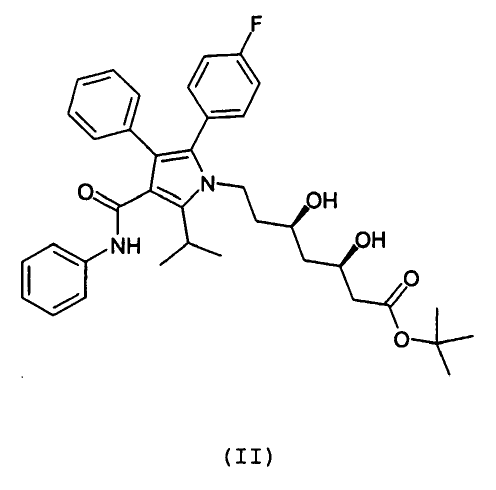 which in the following is referred to as atorvastatin tert.-butyl ester (II).

The invention also relates to a process for preparation of these crystalline forms which uses as starting material (4R-cis)-6-{2-[2-(4-fluorophenyl)-5-(1-methylethyl)-3-phenyl-4-[(phenylamino)carbonyl]pyrrole-1-yl]ethyl}-2,2-dimethyl-1,3-dioxane-4-acetic acid tertiary butyl ester of formula (I) 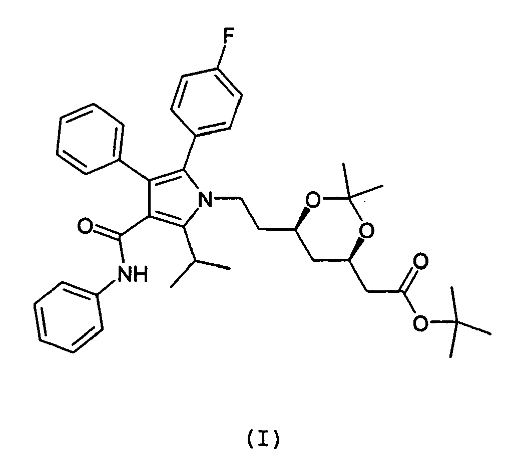 and which in the following is referred to as dimethyl ketal of atorvastatin tert.-butyl ester (I).

More particularly the invention relates to the use of these new polymorphs as intermediates for the preparation of pharmaceutically acceptable salts of atorvastatin.

### Background of the Invention

Atorvastatin tert.-butyl ester (II) is a known and valuable precursor for the preparation of the HMG-CoA reductase inhibitor atorvastatin ([R-(R*,R*)]-2-(4-fluorophenyl)-β,δ-dihydroxy-5-(1-methylethyl)-3-phenyl-4-[(phenylamino)carbonyl]-1*H-*pyrrole-1-heptanoic acid) and its salts and pharmaceutically acceptable esters, all of which are members of the class of drugs called statins.

Statins suppress cholesterol biosynthesis by competitively inhibiting 3-hydroxy-3-methyl-glutaryl-coenzyme A reductase which catalyzes the conversion of HMG-CoA to mevalonate, which is the rate determining step in the biosynthesis of cholesterol. They are currently the most therapeutically effective drugs available for the treatment of hyperlipidemia and hypercholesterolemia, both of which are risk factors for arteriosclerosis and coronary heart disease. Processes for the preparation of atorvastatin and key intermediates are disclosed in, for example, the United States Patents No.: 5,003,080; 5,097,045; 5,103,024; 5,124,482; 5,149,837; 5,155,251; 5,216,174; 5,245,047; 5,248,793; 5,280,126; 5,342,952, 5,397,792; 4,681,893; 5,273,995, and 5,298,627.

Further, K.L. Baumann et al. describe in Tetrahedron Lett. 1992, 33, 2283-2284 the preparation of dimethyl ketal of atorvastatin tert.-butyl ester (I) by a Paal-Knorr pyrrole synthesis using a ternary solvent mixture of toluene-heptane-tetrahydrofuran (1:4:1) with catalysis by pivalic acid and conversion of (I) to atorvastatin hemi-calcium without isolating any intermediates. A similar procedure was reported by Peter W.K. Woo et al. in J. Label. Compd. Radiopharm., 1999, vol. 42, part II, 135-145 and by B.-C. Chen et al. in J. Label. Compd. Radiopharm., 2000, vol. 43, part III, 261-270.

Also WO 02/059087 describes the direct conversion of the dimethyl ketal of atorvastatin tert.-butyl ester (I) into non-crystalline atorvastatin hemi-calcium or atorvastatin lactone without isolating intermediary occuring products.

H. T. Lee et al. describe in J. Label. Compd. Radiopharm., 1999, vol. 42, part II, 129-133 the preparation of the atorvastatin tert.-butyl ester (II) by reduction of the corresponding β-ketoester.

WO 02/083637 and WO 02/083638 disclose that the treatment of dimethyl ketal of atorvastatin tert.-butyl ester (I) in methanol with aqueous hydrochloric acid yielded a complicated mixture containing 5 intermediates, as revealed by HPLC analysis. The percentage and distribution of the detected compounds in the reaction mixture varied depending on the reaction conditions. However, none of the intermediates, including atorvastatin tert.-butyl ester (II), was isolated, but the reaction solution was further treated with dilute aqueous sodium hydroxide solution.

Furthermore, WO 02/43667 and WO 03/016317 also reported difficulties during the preparation of atorvastatin hemi-calcium, in particular during the step of converting dimethyl ketal of atorvastatin tert.-butyl ester (I) using an acid catalyst to atorvastatin tert.-butyl ester (II). The product proved to contain other compounds, such as atorvastatin lactone and atorvastatin free acid, and was isolated e.g. as an oil which indicates that atorvastatin tert.-butyl ester (II) prepared in this manner is not substantially pure. Thus, these processes were not able to provide atorvastatin tert.-butyl ester (II) in a well defined crystalline form of high purity.

The above shows that the processes of the prior art are not capable of producing atorvastatin tert.-butyl ester (II) in crystalline form and therefore a form of high purity.

Moreover, these prior art processes do not lead to atorvastatin hemi-calcium having a filterability and purity which is satisfactory for the preparation of pharmaceutical formulations as impurities are present, which are hard to remove.

Consequently, there is still a need for an improved process to prepare atorvastatin, in particular in form of its hemi-calcium salt with good filterability and high purity making it very well suitable for the preparation of pharmaceutical formulations.

Further, there is a need for precursors which allow the easy preparation of polymorphic forms of atorvastatin and its pharmaceutically acceptable salts or the conversion to other forms of atorvastatin and its pharmaceutically acceptable salts.

These problems are solved by the present invention.

### Detailed Description of the Invention

First of all, the invention relates to two well defined polymorphic forms of atorvastatin tert.-butyl ester (II) designated as crystalline form 1 and form 2, which are excellent intermediates for the preparation of atorvastatin and derivatives thereof, in particular for the preparation of amorphous atorvastatin hemi-calcium. These two polymorphic forms were
characterized by means of X-ray analysis and differential scanning calorimetry.

X-ray powder diffraction patterns were obtained by a Phillips PW3040/60 X'Pert PRO diffractometer using CuK_{α} radiation of 1,541874Å.

DSC curves were recorded on a Perkin Elmer DSC 7 Scanning calorimeter. Samples of approx. 3 mg were scanned at a heating rate of 10°C/min under nitrogen atmosphere in open aluminium DSC pans.

The crystalline form 1 of atorvastatin tert.-butyl ester (II) according to the invention is characterized by an X-ray powder diffraction pattern having peaks at: 6.10, 6.83, 10.77, 17.01 ± 0.1 degrees two-theta.

It is preferably characterized by an X-ray powder diffraction pattern having peaks at: 6.10, 6.83, 10.77, 12.18, 16.20, 17.01, 17.65, 19.07, 19.80, 21.25, 21.64, 23.70, 27.60 ± 0.1 degrees two-theta.

A typical X-ray diffraction pattern of crystalline form 1 is given in the following Table 1 by listing 2-theta degrees together with the corresponding intensities. In the table "s" designates a strong relative intensity from 30 to 100 % and "m" designates a medium relative intensity from 10 to 30 %.

**Table 1:**

| **2**Θ | **Intensity** |
|---|---|
| 6.10 | s |
| 6.83 | s |
| 10.77 | s |
| 12.18 | m |
| 16.20 | m |
| 17.01 | s |
| 17.65 | m |
| 19.07 | m |
| 19.80 | m |
| 21.25 | m |
| 21.64 | m |
| 23.70 | m |
| 27.60 | m |

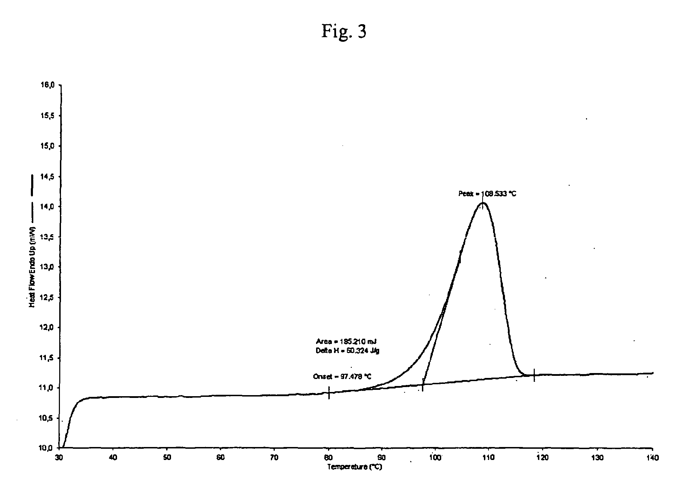

Further, the crystalline form 1 of atorvastatin tert.-butyl ester (II) according to the invention is characterized by the DSC curve shown in Figure 3 with the onset temperature at about 97 °C.

The crystalline form 2 of atorvastatin tert.-butyl ester (II) according to the invention is characterized by an X-ray powder diffraction pattern having peaks at: 6.48, 12.15, 17.21, 18.34, 20.18, 20.47, 24.45 ± 0.1 degrees two-theta.

It is preferably characterized by an X-ray powder diffraction pattern having peaks at: 6.48, 7.59, 10.97, 12.15, 15.02, 17.21, 18.34, 20.18, 20.47, 21.59, 24.45, 26.07, 29.41 ± 0.1 degrees two-theta.

A typical X-ray diffraction pattern of crystalline form 2 is given in the following Table 2 by listing 2-theta degrees together with the corresponding intensities. In the table "s" designates a strong relative intensity from 30 to 100 % and "m" designates a medium relative intensity from 10 to 30 %.

**Table 2:**

| **2**Θ | **Intensity** |
|---|---|
| 6.48 | s |
| 7.59 | m |
| 10.97 | m |
| 12.15 | s |
| 15.02 | m |
| 17.21 | s |
| 18.34 | s |
| 20.18 | s |
| 20.47 | s |
| 21.59 | m |
| 24.45 | s |
| 26.07 | m |
| 29.41 | m |

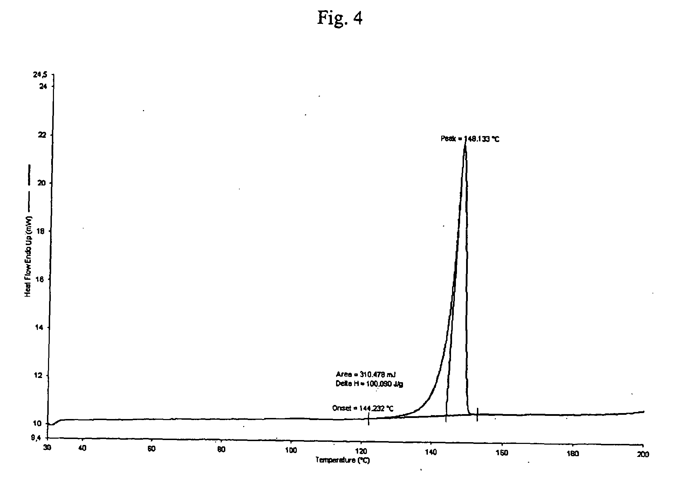

Further, the crystalline form 2 of atorvastatin tert.-butyl ester (II) according to the invention is characterized by the DSC curve shown in Figure 4 with the onset temperature at about 144 °C.

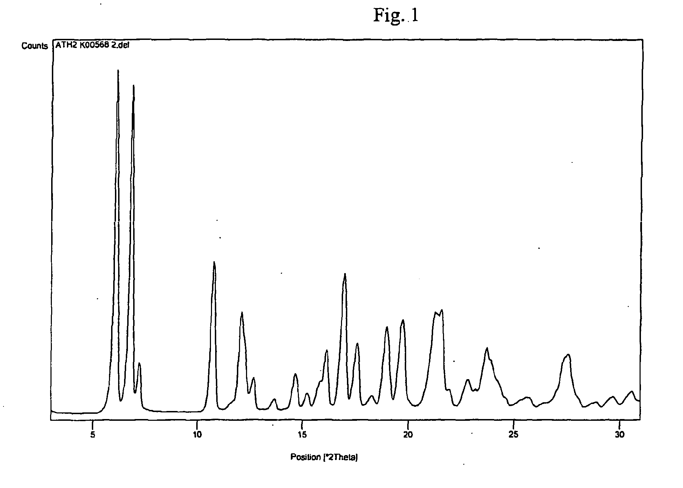

Fig. 1 shows the X-ray powder diffraction pattern of form 1 of atorvastatin tert.-butyl ester (II).

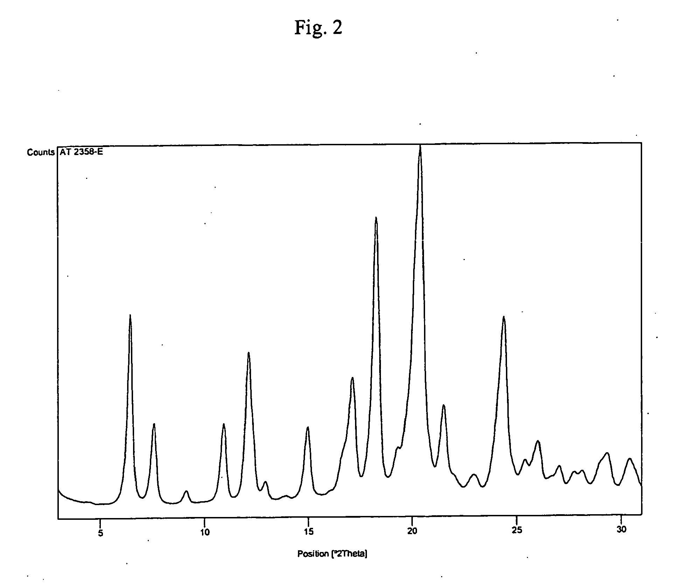

Fig. 2 shows the X-ray powder diffraction pattern of form 2 of atorvastatin tert.-butyl ester (II).
Fig. 3 shows the DSC curve of form 1 of atorvastatin tert.-butyl ester (II).
Fig. 4 shows the DSC curve of form 2 of atorvastatin tert.-butyl ester (II).

The crystalline form 1 of atorvastatin tert.-butyl ester (II) according to the invention is prepared by a process which comprises dissolving any form of dimethyl ketal of atorvastatin tert.-butyl ester (I) in a water miscible solvent and adding an aqueous solution of acid at a temperature from 10 to 50°C, preferably at a temperature from 10 to 40°C and most preferably at room temperature.

The crystalline form 2 of atorvastatin tert.-butyl ester (II) according to the invention is prepared by a process which comprises dissolving any form of dimethyl ketal of atorvastatin tert.-butyl ester (I) in a water miscible solvent and adding an aqueous solution of acid at a temperature from 50 to 100°C, preferably at reflux of the mixture.

In the processes for preparing form 1 and form 2, the dimethyl ketal of atorvastatin tert.-butyl ester (I) used as starting material can be in any form, e.g. it can be present in a reaction solution, in a filtrate, in any crude, polymorphic, anhydrous, solvated, or hydrated form, or mixtures thereof, prepared following any of the processes known to the expert or described in prior art.

The water miscible solvent is preferably an organic solvent and in particular acetonitrile, 1,2-dimethoxyethane, tetrahydofuran, a lower alcohol, such as methanol, ethanol, propanol, isopropanol, and butanol, or ethyl acetate. The solvent is most preferably acetonitrile. Further, the acid is preferably HCl.

It is also preferred that in the preparation of forms 1 and 2 of atorvastatin tert.-butyl ester (II) the water miscible solvent and the water solution of acid are used in a ratio of 10 to 1, more preferably 6 to 1 and most preferred 4 to 1 parts by volume.

Moreover, it is advantageous to carry out the reaction of dimethyl ketal of atorvastatin tert.-butyl ester (II) with the acid at the given temperature for 3 to 24 h. This reaction time is sufficient to conclude the reaction.

Finally, it has also proved beneficial to add water to the mixture after conclusion of the reaction in order to precipitate the atorvastatin tert.-butyl ester. It is preferred that the water is added in a volume which is higher than that of the water miscible solvent and more preferably higher than the volume of the total reaction mixture.

It is surprising that this specific precipitation step leads to a product which can be separated in a high yield and a high purity. This is a substantial advantage compared to the prior art processes which lead to complicated reaction mixtures.

By the processes according to the invention, it was possible to substantially reduce persistent impurities. Thus, the purity of the obtained atorvastatin tert.-butyl ester (II) is usually higher than 98%, preferably higher than 99%.

In another preferred embodiment, it is also possible to prepare the crystalline form 2 of atorvastatin tert.-butyl ester (II) by crystallization of atorvastation tert.-butyl ester (II) from a specific solvent selected from ethyl acetate, methanol, ethanol, acetonitrile and isopropanol. The solvent is preferably acetonitrile or isopropanol.

The crystalline forms 1 and 2 according to the invention are ideally suited as intermediates for the preparation of any atorvastatin form, e.g. atorvastatin free acid, atorvastatin lactone, or an atorvastatin salt, or of atorvastatin derivatives.

Preferably the crystalline forms 1 and 2 are used for the preparation of any form of an atorvastatin salt in its amorphous or any polymorphic form, and most preferably for the preparation of amorphous atorvastatin hemi-calcium.

The invention therefore also relates to a process for the preparation of any atorvastatin form, preferably of atorvastatin hemi-calcium, by using the crystalline form 1 or the crystalline form 2.

Preferably the process comprises
reacting crystalline form 1 or crystalline form 2 with a base in a mixture of tert.-butyl methyl ether, methanol and water, wherein the volume of water is higher than the volume of tert.-butyl methyl ether,
adding a calcium salt to effect formation of atorvastatin hemi-calcium, and
adding water to the reaction mixture to effect precipitation of atorvastatin hemi-calcium.

The base is in particular NaOH, and the calcium salt is in particular calcium chloride or calcium acetate.

It has surprisingly been shown that the process according to the invention eliminates the drawbacks of the known procedures and provides a simple, reproducible and economically feasible method for the preparation of highly pure and uniformly amorphous atorvastatin hemi-calcium. Morover, the process yields in a product which is extremely well filterable due to the large size of the precipitated particles. This is a substantial benefit in separating the product and may also be responsible for the high yield and purity of the product.

Thus, the present invention provides the conversion of the dimetyl ketal of atorvastatin tert.-butyl ester (I) into a highly pure form of the atorvastatin tert.-butyl ester (II) and conversion of the latter into highly pure atorvastatin hemi-calcium in non-crystalline, in particular amorphous form.

In a further aspect, the present invention also provides a process for the preparation of a pharmaceutical formulation containing highly pure atorvastatin hemi-calcium which has been prepared from crystalline forms 1 and 2 of atorvastatin tert.-butyl ester (II) according to the invention in the non-crystalline, in particular in the amorphous form, of atorvastatin hemi-calcium.

The invention is in the following illustrated further by means of Examples.

### Examples

### Preparation of form 1 of [R-(R*,R*)]-2-(4-fluorophenyl)-β,δ-dihydroxy-5-(1-methylethyl)-3-phenyl-4-[(phenylamino)-carbonyl]-1H-pyrrole-1-heptanoic acid tertiary butyl ester (II) by condnesation of 4-fluoro-α-[2-methyl-1-oxopropyl]-γ-oxo-N,β-diphenylbenzenebutaneamide and (4R-cis)-6-(2-aminoethyl)-2,2-dimethyl-1,3-dioxane-4-acetic acid tertiary butyl ester

### Example 1

To a solution of 1.32 g of (4R-cis)-6-(2-aminoethyl)-2,2-dimethyl-1,3-dioxane-4-acetic acid tertiary butyl ester (4.8 mmol, 1.2 equiv.) in a 9: 1 mixture of heptane and toluene (30 mL), 0.49 g of 2-methylbutyric acid (0.52 mL, 4.8 mmol, 1.2 equiv.) and 1.66 g of 4-fluoro-α-[2-methyl-1-oxopropyl]-γ-oxoN,β-diphenylbenzenebutaneamide (4.0 mmol) are consecutively added. The heterogeneous mixture is stirred at reflux under an argon atmosphere for 22 h. The resulting yellow solution is allowed to cool to room temperature, diluted with 30 mL of tert.-butyl methyl ether and washed consecutively with 50 mL of 1M NaOH, 30 mL of 1M HCl and finally with brine. Evaporation of solvents results in a viscous light brown colored residue, which is dissolved in 12 mL of acetonitrile, 2.2 mL of water and 0.6 mL of 1M HCl. The resulting clear solution is stirred overnight, during that period some precipitation occurs. 10 mL of water is added followed by 20 mL acetonitrile, the latter being preferably used to bring a semisolid precipitate into a filterable one. The stirring is continued for at least 1h. The solid is filtered off, the air-dried material is crystallized twice from acetonitrile and the product is vacuum dried at 20-25 °C till constant weight.
0.70 g, 28.5 % of crystallized tertiary butyl ester of [R-(R*,R*)]-2-(4-fluorophenyl)-β,δ-dihydroxy-5-(1-methylethyl)-3-phenyl-4-[(phenylamino)carbonyl]-1*H*-pyrrole-1-heptanoic acid (II) is isolated having a melting range of 90-96 °C. The purity of the obtained product is about 96 %.

### Example 2

To a solution of 1.32 g (4R-cis)-6-(2-aminoethyl)-2,2-dimethyl-1,3-dioxane-4-acetic acid tertiary butyl ester (4.8 mmol) in a 9:1 mixture of heptane and toluene (30 mL), 0.245 g of 2-methylbutyric acid (0.26 mL, 2.4 mmol) and 1.66 g of 4-fluoro-α-[2-methyl-1-oxopropyl]-γ-oxo-N,β-diphenylbenzenebutaneamide (4.0 mmol) are consecutively added. The heterogeneous mixture is stirred at reflux under an argon atmosphere for 48 h. The resulting yellow solution is allowed to cool to room temperature, diluted with 30 mL of tert-butyl methyl ether and washed consecutively with 50 mL of 1M NaOH, 30 mL of 1M HCl and finally with brine. Evaporation of solvents results in a viscous orange colored residue, which is dissolved in 12 mL of acetonitrile, 2.2 mL of water and 0.6 mL of 1M HCl. The resulting mixture is heated at 45-49 °C with stirring for 5.5 h, until the consumption of the intermediate of formula (I) is found to be almost complete according to HPLC analysis. The mixture is allowed to cool and the stirring is continued at 20-25 °C for 2 h, during that period some precipitation took place. 10 mL of water is added followed by 10 mL of acetonitrile, the latter being preferably used to bring a semisolid precipitate into a filterable slurry and the stirring is continued for about 1h. The solid is filtered off, the filter cake is washed with 4 mL of 50% (v/v) aqueous acetonitrile and the product is vacuum dried at 20-25 °C till constant weight (0.754 g). The remaining filtrate is stirred at room temperature overnight. A second crop of solid is filtered off, washed with 4 mL of 50% (v/v) aqueous acetonitrile and the product is vacuum dried at 20-25 °C till constant weight (0.373 g), which is of identical quality to the first crop according to HPLC analysis. 1.127 g, 45.8% of crystallized tertiary butyl ester of [R-(R*,R*)]-2-(4-fluorophenyl)-β,δ-dihydroxy-5-(1-methylethyl)-3-phenyl-4-[(phenylamino)carbonyl]-1*H*-pyrrole-1-heptanoic acid (II) is isolated having a melting range of 84-91 °C. The purity of the obtained product is higher than 98 %.

### Preparation of form I of [R-(R*,R*)]-2-(4-fluorophenyl)-β,δ-dihydroxy-5-(1-methylethyl)-3-phenyl-4-[(phenylamino)-carbonyl]-1H-pyrrole-1-heptanoic acid tertiary butyl ester (II)

### Example 3

3 g, 4.58 mmol of (4R-cis)-6-{2-[2-(4-fluorophenyl)-5-(1-methylethyl)-3-phenyl-4-[(phenylamino)carbonyl]pyrrole-1-yl]-ethyl}-2,2-dimethyl-1,3-dioxane-4-acetic acid tertiary butyl ester (I) is added to a 50 mL reaction flask and suspended in 13.5 mL of acetonitrile, 2.6 mL of water and 0.72 mL of 1M HCl. The reaction mixture is stirred for at least 12h, 30 mL of water was added and the stirring is continued for at least 1h. The solid precipitate is filtered off and the filter cake is washed with 1 mL of water. The wet cake is dried at 20-25 °C for 3h and at 45-50 °C for 6h till LOD (Loss on drying) <0.5%.

185.8 mg, 98.9 % of [R-(R*,R*)]-2-(4-fluorophenyl)-β,δ-dihydroxy-5-(1-methylethyl)-3-phenyl-4-[(phenylamino)-carbonyl]-1*H*-pyrrole-1-heptanoic acid tertiary butyl ester (II) is isolated having a melting range of 100-102 °C. The purity of the obtained product is higher than 99 %.

### Preparation of form II of [R-(R*,R*)]-2-(4-fluorophenyl)-β,δ-dihydroxy-5-(1-methylethyl)-3-phenyl-9-[(phenylamino)-carbonyl]-1H-pyrrole-1-heptanoic acid tertiary butyl ester (II)

### Example 4

10 g, 15.3 mmol of (4R-cis)-6-{2-[2-(4-fluorophenyl)-5-(1-methylethyl)-3-phenyl-4-[(phenylamino)carbonyl]pyrrole-1-yl]-ethyl}-2,2-dimethyl-1,3-dioxane-4-acetic acid tertiary butyl ester (I) is added to a 500 mL reaction flask and suspended in 45 mL of acetonitrile, 18.8 mL of water and 2.4 mL of 1M HCl. The reaction mixture is heated to 45 °C until all starting material is dissolved and the reaction is followed by HPLC method. After 4 h the reaction mixture is cooled to room temperature, and 100 mL of water is added. The solid precipitate is filtered off and the cake is washed with 50 mL of a solvent mixture comprising acetonitrile and water (1:1, v/v). The wet cake is dried at 20-25 °C on air till constant weight. 8.45 g, 89.9 %, of [R-(R*,R*)]-2-(4-fluorophenyl)-β,δ-dihydroxy-5-(1-methylethyl)-3-phenyl-4-[(phenylamino)-carbonyl]-1*H*-pyrrole-1-heptanoic acid tertiary butyl ester (II) is isolated having a melting range of 142-145 °C. The purity of the obtained product is higher than 99 %.

### Example 5

1 g, 1.5 mmol of [R-(R*,R*)]-2-(4-fluorophenyl)-β,δ-dihydroxy-5-(1-methylethyl)-3-phenyl-4-[(phenylamino)carbonyl]-1*H-*pyrrole-1-heptanoic acid tertiary butyl ester (II) is dissolved in 5 ml of acetonitrile and heated to reflux. The crystallization mixture is slowly cooled to room temperature and the formation of crystals occurs. The thick suspension is cooled to 0-5 °C and the crystals are filtered off. The product is dried at 20-25 °C on air till constant weight.
0.915 g, 91.5 % of crystallized [R-(R*,R*)]-2-(4-fluorophenyl)-β,δ-dihydroxy-5-(1-methylethyl)-3-phenyl-4-[(phenylamino)carbonyl]-1*H*-pyrrole-1-heptanoic acid tertiary butyl ester (II) is isolated having a melting range of 146-148 °C. The purity of the obtained product is higher than 99%.

### Example 6

1 g, 1.5 mmol of [R-(R*,R*)]-2-(4-fluorophenyl)-β,δ-dihydroxy-5-(1-methylethyl)-3-phenyl-4-[(phenylamino)carbonyl]-1*H-*pyrrole-1-heptanoic acid tertiary butyl ester (II) is dissolved in 10 ml of 2-propanol at boiling temperature of the solvent. The crystallization mixture is slowly cooled down to room temperature and then cooled to 0-5 °C. Formed crystals are filtered off. The product is dried at 20-25 °C on air till constant weight.
0.626g, 62.6 % of crystallized product is isolated having a melting range of 141-143°C. The purity of the obtained product is higher than 99 %.

### Preparation of amorphous atorvastatin hemi-calcium

### Example 7

To a solution of 1 g of [R-(R*,R*)]-2-(4-fluorophenyl)-β,δ-dihydroxy-5-(1-methylethyl)-3-phenyl-4-[(phenylamino)-carbonyl]-1*H*-pyrrole-1-heptanoic acid tertiary butyl ester (II) (1.62 mmol) in 3.4 mL of methanol and 2.8 mL of tert-butyl methyl ether, 68.4 mg of NaOH (1.7 mmol, 1.05 equivalent) and 5.8 mL of water are added. The reaction mixture is purged with a nitrogen flow for about 5 minutes and heated to the reflux for 2-4 h, until the concentration of starting [R-(R*,R*)]-2-(4-fluorophenyl)-β,δ-dihydroxy-5-(1-methylethyl)-3-phenyl-4-[(phenylamino)carbonyl]-1*H*-pyrrole-1-heptanoic acid tertiary butyl ester (II) is lower than 0.5%, determined with HPLC method.
The reaction mixture is allowed to cool to 20-25 °C, active charcoal is added, and the reaction mixture is stirred for additional 30 min. The reaction mixture is filtered and the pH of the filtrate is set to 8.0-8.2 by the addition of HCl. The reaction mixture is washed with 3 × 2.9 mL of tert-butyl methyl ether and aqueous phases are finally filtered. The reaction mixture is purged with nitrogen flow for about 5 min and 0.179 g of CaCl₂ (0,82 mmol of CaCl₂·6H₂O) and 6.8 mL of water are added in a 15-20 min interval at 20-25 °C. After the complete addition of CaCl₂, the reaction mixture is stirred for additional 15-30 min. Then 6 mL of water is slowly added into the reaction mixture to provoke the solidification of the thick emulsion-like mixture. Hemi-calcium salt of atorvastatin is formed in the form of particles having a size in the range of mm to several mm which show improved filterability. The precipitate is filtered off after 30 min. The wet cake is washed with water. Collected solid material is dried on air to obtain dry solid atorvastatin hemi-calcium.

### Example 8

To the solution of 9.22 g of [R-(R*,R*)]-2-(4-fluorophenyl)-β,δ-dihydroxy-5-(1-methylethyl)-3-phenyl-4-[(phenylamino)-carbonyl]-1*H*-pyrrole-1-heptanoic acid tertiary butyl ester (II) (15 mmol) in 61 mL of methanol and 52 mL tert-butyl methyl ether, 0.63 g of NaOH (15.7 mmol, 1.05 equivalent) and 104 mL of water are added. The reaction mixture is purged with a nitrogen flow for about 5 minutes and heated to the reflux for 2-4 h, until the concentration of the starting compound [R-(R*,R*)]-2-(4-fluorophenyl)-β,δ-dihydroxy-5-(1-methylethyl)-3-phenyl-4-[(phenylamino)carbonyl]-1*H*-pyrrole-1-heptanoic acid tertiary butyl ester (II) is lower than 0.5 %, determined by HPLC.
The reaction mixture is allowed to cool to 20-25 °C and the pH is set to 8.0-8.2 by the addition of HCl. The reaction mixture is washed with 3 × 26 mL of tert-butyl methyl ether and aqueous phases are finally filtered. The reaction mixture is purged with a nitrogen flow for about 5 min, and 1.80 g of CaCl₂ (8.2 mmol of CaCl₂·6H₂O) and 64 mL of water are added in a 15-20 min interval at 20-25 °C. After the complete addition of CaCl₂, the reaction mixture is stirred for additional 15-30 min and 270 mL of water is slowly added into the reaction mixture to provoke the solidification of the thick emulsion-like mixture. Hemi-calcium salt of atorvastatin is formed in the form of granules, which show improved filterability. The precipitate is filtered off and the wet cake is washed with a mixture of water and methanol and finally with water. The collected solid material is dried on air to obtain dry solid atorvastatin hemi-calcium.

The dry hemi-calcium salt of atorvastatin can optionally be additionally milled on a dry pearl mill.

### Example 9:

To a solution of 5.47 g of (4R-cis)-6-(2-aminoethyl)-2,2-dimethyl-1,3-dioxane-4-acetic acid tertiary butyl ester (20 mmol, 1.0 equiv.) in a 9:1 mixture of heptane and toluene (150 mL) are added consecutively 2.04 g of pivalic acid (2.30 mL, 20 mmol, 1.0 equiv.) and 8.30 g of 4-fluoro-α-[2-methyl-l-oxopropyl]-γ-oxo-N,β-diphenylbenzenebutaneamide (20 mmol). The heterogeneous mixture is stirred at reflux under an argon atmosphere for 25 h. The resulting yellow solution is allowed to cool to room temperature, diluted with 150 mL of tert-butyl methyl ether and washed consecutively with 150 mL of 1M NaOH, 2 x 150 mL of 1M HCl and finally with brine. Evaporation of solvents resulted in a bright yellow colored foam, which is dissolved in 60 mL of acetonitrile, 11 mL of water and 3.0 mL of 1M HCl. The resulting mixture is heated at 45-50 °C with stirring for 6.5 h, until the consumption of the intermediate having formula (I) is found to be almost complete according to HPLC analysis. The mixture is allowed to cool, 50 mL of water is added and the stirring is continued at 20-25 °C for 2 h, during that period some material deposited. Then 50 mL of acetonitrile is added in order to bring a semisolid precipitate into a filterable slurry and the stirring is continued for about 1h. The solid is filtered off, the filter cake is washed with 20 mL of 50% (v/v) aqueous acetonitrile and vacuum dried at 20-25 °C till constant weight. 4.77 g (39 %) of the compound of formula (II) is isolated with melting range 90-95 °C and higher than 98 % purity.

To the solution of 4.75 g of ester (II) (7.73 mmol) in 16.5 mL of methanol and 14 mL of tert-butyl methyl ether, 0.325 g of NaOH (8.1 mmol, 1,05 equiv.) and 29 mL of water are added. The reaction mixture is purged with a nitrogen flow for about 5 minutes, heated to the reflux for 4 h and cooled to room temperature. The pH of solution was adjusted to 11 with 2M NaOH and the reflux continued for 3 h. The reaction mixture is allowed to cool to 20-25 °C, the pH is set to 8.0-8.2 by the addition of HCl. The mixture is washed with 3 × 15 mL of tert-butyl methyl ether and the aqueous phases are finally filtered. The reaction mixture is purged with a nitrogen flow for about 5 minutes, and 0.836 g of CaCl₂·6H₂O (3.82 mmol, 0.99 equiv.) in 19 mL of water is added in a 15-20 min interval at 20-25 °C. After the complete addition of CaCl₂, the reaction mixture is stirred for additional 15-30 min and 80 mL of water is slowly added into the reaction mixture to provoke the solidification of the thick emulsion-like mixture. Hemi-calcium salt of atorvastatin is formed in the form of granules, which show improved filterability. The solid is filtered off after 2 h. The wet cake is washed with a mixture of water and methanol and finally with water. The collected solid material is dried on air to obtain 3.817 g (33% from 4-fluoro-α-[2-methyl-1-oxopropyl]-γ-oxo-N,β-diphenylbenzenebutaneamide) of dry solid atorvastatin hemi-calcium.

## Claims

1. Crystalline form 1 of atorvastatin tert.-butyl ester of the formula (II) 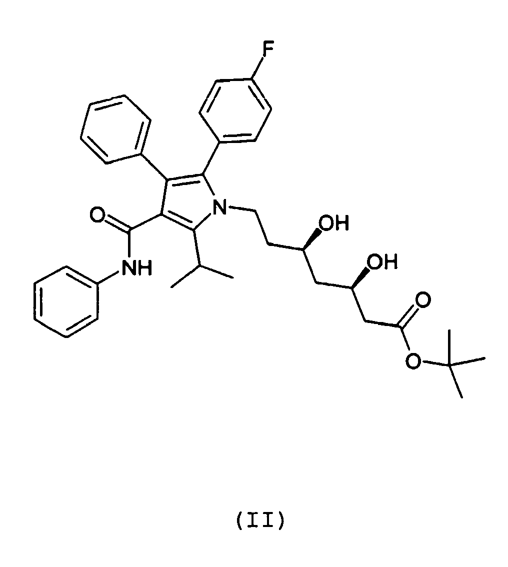 **characterized by** an X-ray powder diffraction pattern having peaks at: 6.10, 6.83, 10.77, 17.01 ± 0.1 degrees two-theta.

2. Crystalline form 1 according to claim 1 which is **characterized by** an X-ray powder diffraction pattern having peaks at: 6.10, 6.83, 10.77, 12.18, 16.20, 17.01, 17.65, 19.07, 19.80, 21.25, 21.64, 23.70, 27.60 ± 0.1 degrees two-theta.

3. Crystalline form 1 of atorvastatin tert.-butyl ester of formula (II) which is **characterized by** the DSC curve shown in Figure 3 with the onset temperature at about 97 °C.

4. Process for the preparation of the crystalline form 1 of any of claims 1 to 3 which comprises
dissolving any form of dimethyl ketal of atorvastatin tert.-butyl ester of formula (I) 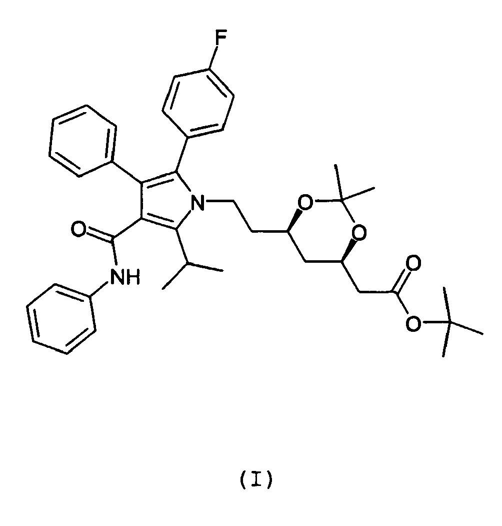 in a water miscible solvent and adding an aqueous solution of acid, preferably HCl, at 10-50 °C, preferably at 10-40 °C, most preferably at room temperature.

5. Process according to claim 4, which further comprises
continuing the reaction of the dimethyl ketal of atorvastatin tert.-butyl ester (I) with the acid for 3 to 24 h at the same temperature to conclude the reaction.

6. Process according to claim 5, which further comprises
adding water to the reaction mixture, after conclusion of the reaction, to precipitate atorvastatin tert.-butyl ester (II).

7. Process according to claim 6, wherein the water is added to the reaction mixture in a volume higher than the volume of the water miscible solvent.

8. Process according to claims 6 or 7, wherein the water is added to the reaction mixture in a volume higher than the volume of the reaction mixture.

9. Process according to any one of claims 4 to 8, wherein the water miscible solvent and the aqueous solution of acid are used in a ratio of 10 to 1, preferably 6 to 1, and most preferably 4 to 1 parts per volume.

10. Process according to any one of claims 4 to 9, wherein the water miscible solvent is acetonitrile.

11. Crystalline form 2 of atorvastatin tert.-butyl ester of the formula (II) 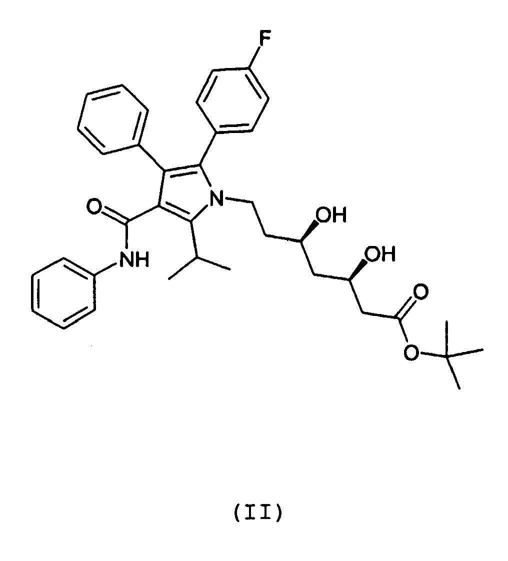 **characterized by** an X-ray powder diffraction pattern having peaks at: 6.48, 12.15, 17.21, 18.34, 20.18, 20.47, 24.45 ± 0.1 degrees two-theta.

12. Crystalline form 2 according to claim 11 which is **characterized by** an X-ray powder diffraction pattern having peaks at: 6.48, 7.59, 10.97, 12.15, 15.02, 17.21, 18.34, 20.18, 20.47, 21.59, 24.45, 26.07, 29.41 ± 0.1 degrees two-theta.

13. Crystalline form 2 of atorvastatin tert.-butyl ester of the formula (II) 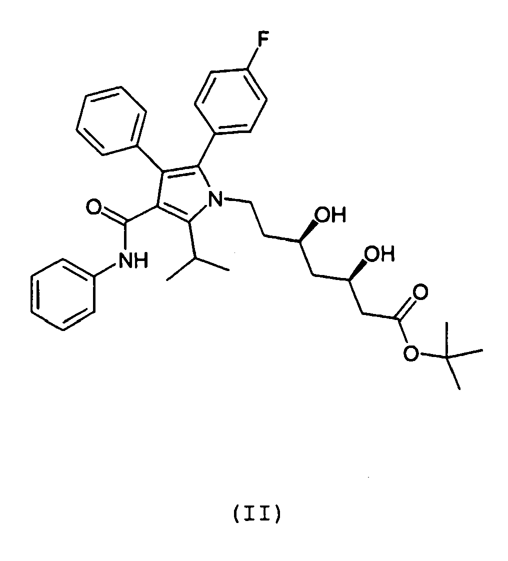 which is **characterized by** the DSC curve shown in Figure 4 with the onset temperature at about 144 °C.

14. Process for the preparation of the crystalline form 2 of atorvastatin tert.-butyl ester according to any one of claims 11 to 13, which comprises
dissolving any form of dimethyl ketal of atorvastatin tert.-butyl ester of formula (I) 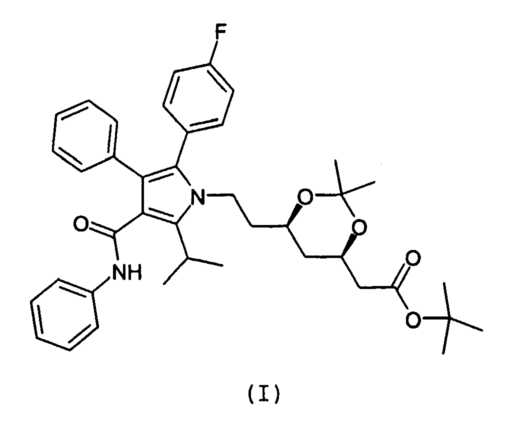 in a water miscible solvent and adding an aqueous solution of acid, preferably HCl, and heating the mixture to 50-100 °C, preferably to the boiling temperature of the mixture.

15. Process according to claim 14, which further comprises
continuing the reaction of the dimethyl ketal of atorvastatin tert.-butyl ester (I) with the acid for 3 to 24 h at the same temperature to conclude the reaction.

16. Process according to claim 15, which further comprises
adding water to the reaction mixture, after conclusion of the reaction, to precipitate atorvastatin tert.-butyl ester (II).

17. Process according to claim 16, wherein the water is added to the reaction mixture in a volume higher than the volume of the water miscible solvent.

18. Process according to claims 16 or 17, wherein the water is added to the reaction mixture in a volume higher than the volume of the reaction mixture.

19. Process according to any one of claims 14 to 18, wherein the water miscible solvent is acetonitrile.

20. Process according to any one of claims 14 to 19, wherein water miscible solvent and the aqueous solution of acid are used in a ratio of 10 to 1, preferably 6 to 1, and most preferably 4 to 1 parts per volume.

21. The use of the crystalline form 1 according to any one of claims 1 to 3, for the preparation of any atorvastatin form, e.g. atorvastatin free acid, atorvastatin lactone, or for the preparation of an atorvastatin salt in amorphous or crystalline form, or a mixture thereof.

22. The use of the crystalline form 2 according to any one of claims 11 to 13 for the preparation of any atorvastatin form, e.g. atorvastatin free acid, atorvastatin lactone, or for the preparation of an atorvastatin salt in amorphous or crystalline form, or a mixture thereof.

23. The use according to claims 21 or 22 for the preparation of atorvastatin hemi-calcium.

24. Process for the preparation of any atorvastatin form, preferably of atorvastatin hemi-calcium, by using the crystalline form 1 according to any one of claims 1 to 3 or the crystalline form 2 according to any one of claims 11 to 13.

25. Process according to claim 24, which comprises
reacting crystalline form 1 or crystalline form 2 with a base in a mixture of tert.-butyl methyl ether, methanol and water, wherein the volume of water is higher than the volume of tert.-butyl methyl ether,
adding a calcium salt to effect formation of atorvastatin hemi-calcium, and
adding water to the reaction mixture to effect precipitation of atorvastatin hemi-calcium.

## Patentansprüche

1. Kristalline Form 1 von Atorvastatin tert.-Butylester der Formel (II) 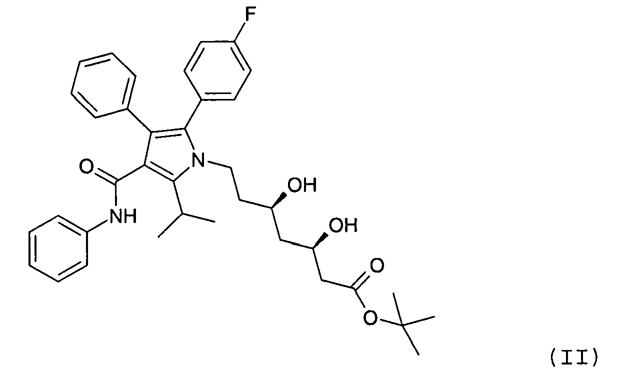 **gekennzeichnet durch** ein Röntgenpulverdiffraktionsmuster, das Peaks bei 6,10, 6,83, 10,77, 17,01 ± 0,1 Grad 2-Theta aufweist.

2. Kristalline Form 1 nach Anspruch 1, welche durch ein Röntgenpulverdiffraktionsmuster **gekennzeichnet** ist, das Peaks bei 6,10, 6,83, 10,77, 12,18, 16,20, 17,01, 17,65, 19,07, 19,80, 21,25, 21,64, 23,70, 27,60 ± 0,1 Grad 2-Theta aufweist.

3. Kristalline Form 1 von Atorvastatin tert.-Butylester der Formel (II), welche durch die in Figur 3 gezeigte DSC Kurve mit der Onset-Temperatur bei etwa 97°C **gekennzeichnet** ist.

4. Verfahren zur Herstellung der kristallinen Form 1 nach einem der Ansprüche 1 bis 3, bei dem
eine beliebige Form des Dimethylketals von Atorvastatin tert.-Butylester der Formel (I) 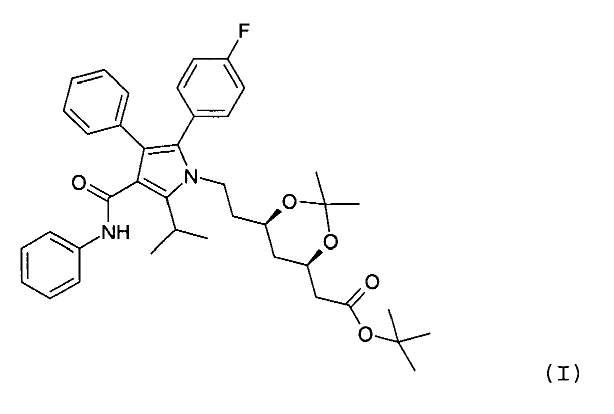 in einem wassermischbaren Lösungsmittel gelöst wird und eine wässrige Säurelösung, vorzugsweise HCl, bei 10 bis 50°C, vorzugsweise bei 10 bis 40°C, besonders bevorzugt bei Raumtemperatur zugegeben wird.

5. Verfahren nach Anspruch 4, bei dem außerdem die Reaktion des Dimethylketals von Atorvastatin tert.-Butylester (I) mit der Säure für 3 bis 24 Stunden bei derselben Temperatur fortgesetzt wird, um die Reaktion abzuschließen.

6. Verfahren nach Anspruch 5, bei dem außerdem nach Abschluss der Reaktion Wasser zu der Reaktionsmischung gegeben wird, um Atorvastatin tert.-Butylester (II) auszufällen.

7. Verfahren nach Anspruch 6, bei dem das Wasser in einem Volumen zu der Reaktionsmischung gegeben wird, das größer ist als das Volumen des wassermischbaren Lösungsmittels.

8. Verfahren nach den Ansprüchen 6 oder 7, bei dem das Wasser in einem Volumen zu der Reaktionsmischung gegeben wird, das größer ist als das Volumen der Reaktionsmischung.

9. Verfahren nach einem der Ansprüche 4 bis 8, bei dem das wassermischbare Lösungsmittel und die wässrige Lösung der Säure in einem Verhältnis von 10 zu 1, vorzugsweise 6 zu 1 und besonders bevorzugt 4 zu 1 Volumenteilen verwendet werden.

10. Verfahren nach einem der Ansprüche 4 bis 9, bei dem das wassermischbare Lösungsmittel Acetonitril ist.

11. Kristalline Form 2 von Atorvastatin tert.-Butylester der Formel (II) 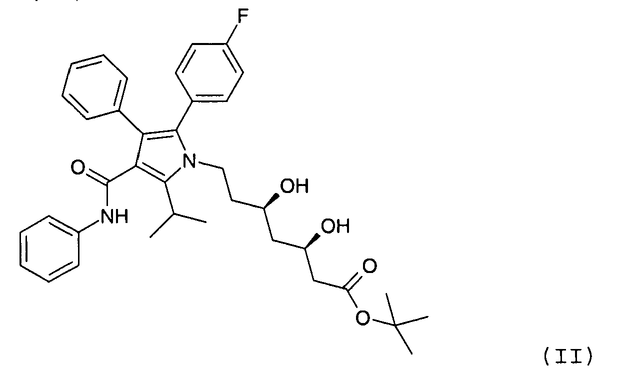 **gekennzeichnet durch** ein Röntgenpulverdiffraktionsmuster, das Peaks bei 6,48, 12,15, 17,21, 18,34, 20,18, 20,47, 24,45 ± 0,1 Grad 2-Theta aufweist.

12. Kristalline Form 2 nach Anspruch 11, welche durch ein Röntgenpulverdiffraktionsmuster **gekennzeichnet** ist, das Peaks bei 6,48, 7,59, 10,97, 12,15, 15,02, 17,21, 18,34, 20,18, 20,47, 21,59, 24,45, 26,07, 29,41 ± 0,1 Grad 2-Theta aufweist.

13. Kristalline Form 2 von Atorvastatin tert.-Butylester der Formel (II) 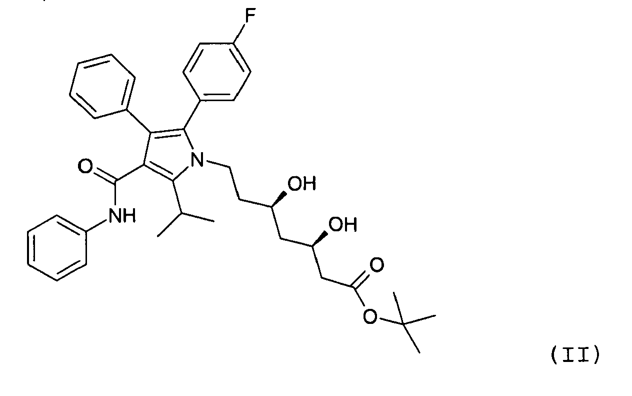 welche durch die in Figur 4 gezeigte DSC Kurve mit der Onsettemperatur bei etwa 144°C **gekennzeichnet** ist.

14. Verfahren zur Herstellung der kristallinen Form 2 von Atorvastatin tert.-Butylester nach einem der Ansprüche 11 bis 13, bei dem
eine beliebige Form des Dimethylketals von Atorvastatin tert.-Butylester der Formel (I) 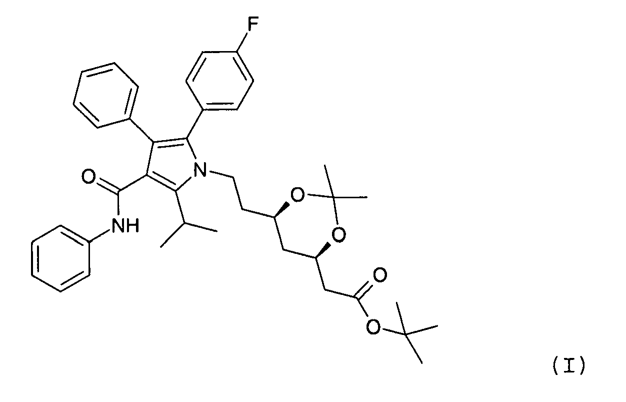 in einem wassermischbaren Lösungsmittel gelöst wird, und eine wässrige Säurelösung, vorzugsweise HCl zugegeben wird und die Mischung auf 50 bis 100°C, vorzugsweise auf die Siedetemperatur der Mischung, erhitzt wird.

15. Verfahren nach Anspruch 14, bei dem außerdem die Reaktion des Dimethylketals von Atorvastatin tert.-Butylester (I) mit der Säure für 3 bis 24 Stunden bei derselben Temperatur fortgesetzt wird, um die Reaktion abzuschließen.

16. Verfahren nach Anspruch 15, bei dem außerdem nach Abschluss der Reaktion Wasser zu der Reaktionsmischung gegeben wird, um Atorvastatin tert.-Butylester (II) auszufällen.

17. Verfahren nach Anspruch 16, bei dem Wasser in einem Volumen zu der Reaktionsmischung gegeben wird, das größer ist als das Volumen des wassermischbaren Lösungsmittels.

18. Verfahren nach den Ansprüchen 16 oder 17, bei dem das Wasser in einem Volumen zu der Reaktionsmischung gegeben wird, das größer ist als das Volumen der Reaktionsmischung.

19. Verfahren nach einem der Ansprüche 14 bis 18, bei dem das wassermischbare Lösungsmittel Acetonitril ist.

20. Verfahren nach einem der Ansprüche 14 bis 19, bei dem das wassermischbare Lösungsmittel und die wässrige Säurelösung in einem Verhältnis von 10 zu 1, vorzugsweise 6 zu 1, und besonders bevorzugt 4 zu 1 Volumenteilen verwendet werden.

21. Verwendung der kristallinen Form 1 nach einem der Ansprüche 1 bis 3 zur Herstellung einer beliebigen Atorvastatinform, z.B. Atorvastatin freie Säure, Atorvastatinlacton, oder zur Herstellung eines Atorvastatinsalzes in amorpher oder kristalliner Form, oder einer Mischung davon.

22. Verwendung der kristallinen Form 2 nach einem der Ansprüche 11 bis 13 zur Herstellung einer beliebigen Atorvastatinform, z.B. Atorvastatin freie Säure, Atorvastatinlacton, oder zur Herstellung eines Atorvastatinsalzes in amorpher oder kristalliner Form, oder einer Mischung davon.

23. Verwendung nach den Ansprüche 21 oder 22 zur Herstellung von Atorvastatinhemicalcium.

24. Verfahren zur Herstellung einer beliebigen Atorvastatinform, vorzugsweise von Atorvastatinhemicalcium, bei dem die kristalline Form 1 nach einem der Ansprüche 1 bis 3 oder die kristalline Form 2 nach einem der Ansprüche 11 bis 13 verwendet wird.

25. Verfahren nach Anspruch 24, bei dem
die kristalline Form 1 oder die kristalline Form 2 mit einer Base in einer Mischung von tert.-Butylmethylether, Methanol und Wasser umgesetzt wird, wobei das Volumen des Wassers größer ist als das Volumen von tert.-Butylmethylether,
ein Calciumsalz zugegeben wird, um die Bildung von Atorvastatinhemicalcium zu bewirken, und
Wasser zu der Reaktionsmischung zugegeben wird, um Fällung von Atorvastatinhemicalcium zu bewirken.

## Revendications

1. Forme cristalline n° 1 de l'ester tertiobutylique d'atorvastatine, de formule (II) : 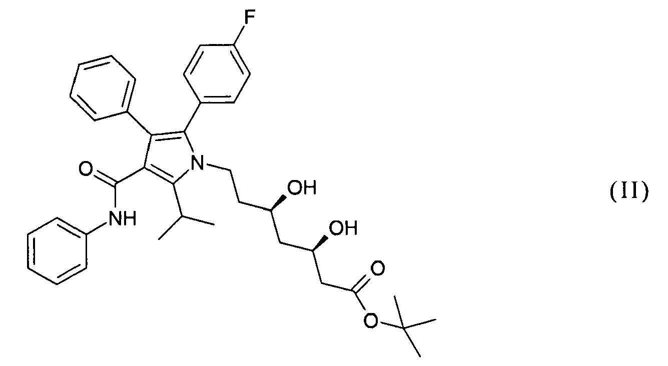 **caractérisée par** un diagramme de diffraction de rayons X par poudre qui présente des pics pour les valeurs suivantes de l'angle 2θ, exprimées en degrés à ± 0,1° près : 6,10, 6,83, 10,77, 17,01.

2. Forme cristalline n° 1 conforme à la revendication 1, **caractérisée par** un diagramme de diffraction de rayons X par poudre qui présente des pics pour les valeurs suivantes de l'angle 2θ, exprimées en degrés à ± 0,1° près : 6,10, 6,83, 10,77, 12,18, 16,20, 17,01, 17,65, 19,07, 19,80, 21,25, 21,64, 23,70, 27,60.

3. Forme cristalline n° 1 de l'ester tertiobutylique d'atorvastatine, de formule (II), conforme à la revendication 1, **caractérisée par** la courbe d'analyse enthalpique différentielle représentée sur la figure 3, présentant une température de début de montée d'environ 97 °C.

4. Procédé de préparation de la forme cristalline n° 1, conforme à l'une des revendications 1 à 3, lequel procédé comporte les étapes suivantes :
dissoudre, dans un solvant miscible à l'eau, de n'importe quelle forme du diméthyl-acétal d'ester tertiobutylique d'atorvastatine, de formule (I) : 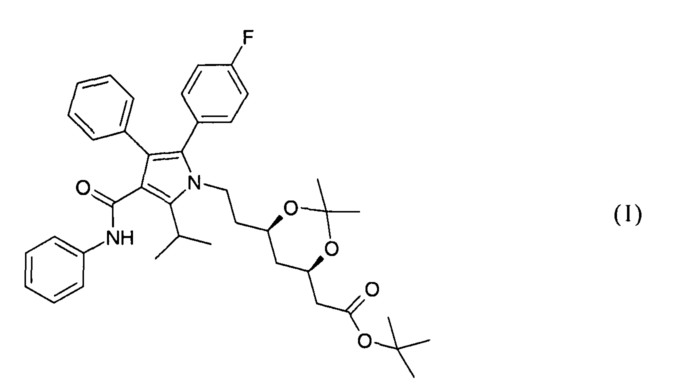
et y ajouter une solution aqueuse d'un acide, de préférence d'acide chlorhydrique, à une température de 10 à 50 °C, de préférence de 10 à 40 °C, et au mieux à température ambiante.

5. Procédé conforme à la revendication 4, qui comporte en outre le fait de laisser la réaction de l'acide et du diméthyl-acétal d'ester tertiobutylique d'atorvastatine de formule (I) se poursuivre à la même température pendant 3 à 24 heures, et aller ainsi jusqu'à son terme.

6. Procédé conforme à la revendication 5, qui comporte en outre le fait d'ajouter de l'eau au mélange réactionnel, une fois la réaction terminée, afin de faire précipiter l'ester tertiobutylique d'atorvastatine de formule (II).

7. Procédé conforme à la revendication 6, dans lequel on ajoute au mélange réactionnel un volume d'eau supérieur au volume du solvant miscible à l'eau.

8. Procédé conforme à la revendication 6 ou 7, dans lequel on ajoute au mélange réactionnel un volume d'eau supérieur au volume du mélange réactionnel.

9. Procédé conforme à l'une des revendications 4 à 8, dans lequel on utilise le solvant miscible à l'eau et la solution aqueuse d'acide en un rapport de parties en volume de 10 à 1, de préférence de 6 à 1 et au mieux de 4 à 1.

10. Procédé conforme à l'une des revendications 4 à 9, dans lequel le solvant miscible à l'eau est de l'acétonitrile.

11. Forme cristalline n° 2 de l'ester tertiobutylique d'atorvastatine, de formule (II) : 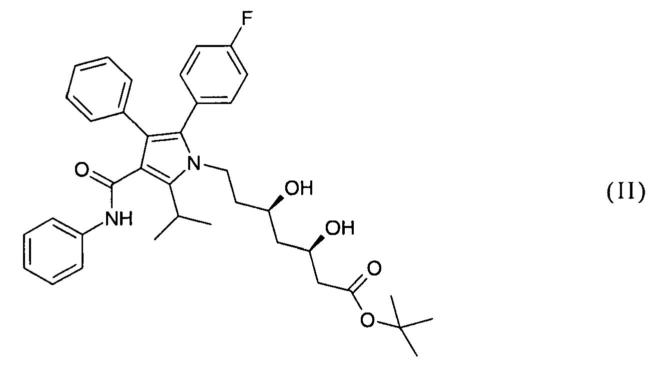 **caractérisée par** un diagramme de diffraction de rayons X par poudre qui présente des pics pour les valeurs suivantes de l'angle 2θ, exprimées en degrés à ± 0,1° près: 6,48, 12,15, 17,21, 18,34, 20,18, 20,47, 24,45.

12. Forme cristalline n° 2 conforme à la revendication 11, **caractérisée par** un diagramme de diffraction de rayons X par poudre qui présente des pics pour les valeurs suivantes de l'angle 2θ, exprimées en degrés à ± 0,1° près : 6,48, 7,59, 10,97, 12,15, 15,02, 17,21, 18,34, 20,18, 20,47, 21,59, 24,45, 26,07, 29,41.

13. Forme cristalline n° 2 de l'ester tertiobutylique d'atorvastatine, de formule (II) : 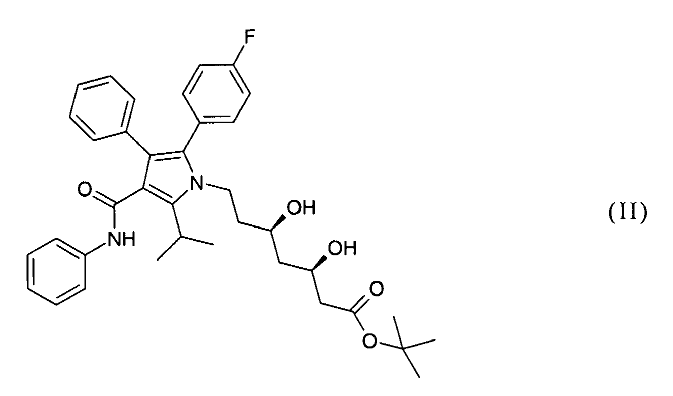 **caractérisée par** la courbe d'analyse enthalpique différentielle représentée sur la figure 4, présentant une température de début de montée d'environ 144 °C.

14. Procédé de préparation de la forme cristalline n° 2 de l'éther tertiobutylique d'atorvastatine, conforme à l'une des revendications 11 à 13, lequel procédé comporte les étapes suivantes :
dissoudre, dans un solvant miscible à l'eau, de n'importe quelle forme du diméthyl-acétal d'ester tertiobutylique d'atorvastatine, de formule (I) : 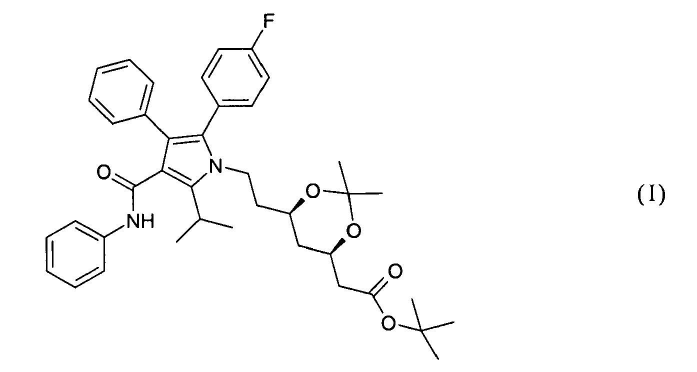
et y ajouter une solution aqueuse d'un acide, de préférence d'acide chlorhydrique, et chauffer le mélange à une température de 50 à 100 °C, et de préférence à la température d'ébullition du mélange.

15. Procédé conforme à la revendication 14, qui comporte en outre le fait de laisser la réaction de l'acide et du diméthyl-acétal d'ester tertiobutylique d'atorvastatine de formule (I) se poursuivre à la même température pendant 3 à 24 heures, et aller ainsi jusqu'à son terme.

16. Procédé conforme à la revendication 15, qui comporte en outre le fait d'ajouter de l'eau au mélange réactionnel, une fois la réaction terminée, afin de faire précipiter l'ester tertiobutylique d'atorvastatine de formule (II).

17. Procédé conforme à la revendication 16, dans lequel on ajoute au mélange réactionnel un volume d'eau supérieur au volume du solvant miscible à l'eau.

18. Procédé conforme à la revendication 16 ou 17, dans lequel on ajoute au mélange réactionnel un volume d'eau supérieur au volume du mélange réactionnel.

19. Procédé conforme à l'une des revendications 14 à 18, dans lequel le solvant miscible à l'eau est de l'acétonitrile.

20. Procédé conforme à l'une des revendications 14 à 19, dans lequel on utilise le solvant miscible à l'eau et la solution aqueuse d'acide en un rapport de parties en volume de 10 à 1, de préférence de 6 à 1, et au mieux de 4 à 1.

21. Utilisation de la forme cristalline n° 1, conforme à l'une des revendications 1 à 3, en vue de la préparation de n'importe quelle forme d'atorvastatine, par exemple l'acide libre atorvastatine ou la lactone d'atorvastatine, ou en vue de la préparation d'un sel d'atorvastatine sous forme amorphe ou cristallisée, ou d'un mélange de telles formes ou de tels sels.

22. Utilisation de la forme cristalline n° 2 conforme à l'une des revendications 11 à 13, en vue de la préparation de n'importe quelle forme d'atorvastatine, par exemple l'acide libre atorvastatine ou la lactone d'atorvastatine, ou en vue de la préparation d'un sel d'atorvastatine sous forme amorphe ou cristallisée, ou d'un mélange de telles formes ou de tels sels.

23. Utilisation conforme à la revendication 21 ou 22, en vue de la préparation du sel hémicalcique d'atorvastatine.

24. Procédé de préparation d'une forme quelconque d'atorvastatine, de préférence du sel hémicalcique d'atorvastatine, dans lequel on utilise de la forme cristalline n° 1, conforme à l'une des revendications 1 à 3, ou de la forme cristalline n° 2, conforme à l'une des revendications 11 à 13.

25. Procédé conforme à la revendication 24, comportant les étapes suivantes :
- faire réagir la forme cristalline n° 1 ou la forme cristalline n° 2 avec une base, dans un mélange de tertiobutyl-méthyl-éther, de méthanol et d'eau dans lequel le volume d'eau est supérieur au volume de tertiobutyl-méthyl-éther,
- ajouter un sel de calcium, pour faire se former le sel hémicalcique d'atorvastatine,
- et ajouter de l'eau au mélange réactionnel, pour faire précipiter le sel hémicalcique d'atorvastatine.
